# EUROPEAN PATENT APPLICATION

(11) **EP 2 609 933 A1**
(43) Date of publication of application: **03.07.2013**
(21) Application number: 11820068.2
(22) Date of filing: 29.08.2011
(51) Int. Cl.: A61K 45/00, A61K 9/08, A61K 31/138, A61K 31/353, A61K 31/4704, A61K 31/5377, A61K 47/10, A61K 47/26, A61P 27/06

(54) **AQUEOUS COMPOSITION FOR OPHTHALMIC ADMINISTRATION**

(30) Priority: 27.08.2010 JP 2010190675
(71) Applicant: Wakamoto Pharmaceutical Co., Ltd., Chuo-ku Tokyo 103-8330 (JP)
(72) Inventor: TAKASHIMA Mitsuyo, Tokyo 1038330 (JP); TAKATA Mayumi, Tokyo 1038330 (JP); SUZUKI Hidekazu, Tokyo 1038330 (JP)
(74) Representative: Vossius & Partner
(86) International application number: PCT/JP2011/069405
(87) International publication number: WO 2012/026609

(57) **Abstract**

The invention provides an aqueous ophthalmic composition, containing a beta blocker such as timolol, carteolol or the like, and a sugar alcohol such as mannitol, sorbitol or the like, optionally together with boric acid. The composition of the invention can improve the corneal permeability of a drug, so that the dose of the drug can be lowered, for example by decreasing the frequency of application to the eyes. It is therefore expected that the risk of systemic side effects which may be induced by the application of the beta blocker to the eyes, including cardiotoxicity or respiratory toxicity can be reduced. The decrease in the frequency of application of ophthalmic solution can favorably improve the QOL and prevent the decrease of therapeutic effect which may be caused by missing the application to the eyes.

## Description

### [Technical Field]

The present invention relates to an aqueous ophthalmic composition with improved intraocular penetration of a medicinal agent when a composition comprising the medicinal agent and a sugar alcohol is applied to the eyes.

### [Background Art]

Currently, beta blocker ophthalmic solutions represented by timolol maleate, carteolol hydrochloride and nipradilol are widely used as therapeutic agents for glaucoma. However, the above-mentioned medicinal agents generally have such a high water solubility that highly hydrophobic corneal epithelium becomes a barrier to penetration of the medicinal agents. To allow a sufficient amount of medicinal agent to intraocularly penetrate and show the efficacy, therefore, the ophthalmic solution containing higher doses of beta blocker should be administered or frequent application of the ophthalmic solution should be needed. In such a way of administration, the medicament not absorbed intraocularly is bound to pass into whole system and be absorbed there, which is considered to increase a risk of the side effects of beta blockers, that is, severe cardiotoxicity and respiratory toxicity. To locally allow the beta blocker to have more effect of lowering the intraocular pressure apart from the systemic action, and keep the intraocular pressure lowering effect longer, improvements in the intraocular penetration of the medicament and the retention of the medicament in the eyes are desirable instead of application of the ophthalmic solution containing higher doses of beta blocker or the ophthalmic solution containing usual dose of beta blocker more frequently.

With respect to the beta blocker, once daily administration has already become possible by taking advantage of the improved retentivity of the beta blocker, resulting from sol-gel transition according to the gelling techniques using heat application and pH change (WO 94/23750 A and JP (Sho) 62-181228 A). Specifically, the medical fluid in the form of a composition containing a particular polymer causes gelation when the medical fluid is heated by the effect of the body temperature or the like upon administration of the fluid to the eyes, or when the pH of the medical fluid is changed by mixing with tears serving as a barrier on the surface of an eyeball. However, use of such gelatinous eye drops produces the problems that blurred vision or sticky feeling lasts for several minutes after application of the eye drops, and that special care should be taken for patients using two or more kinds of eye drops.

In light of the above, various studies have been conducted to improve the corneal permeability of beta blockers. By way of example, there is proposed a method of improving the permeability of the beta blocker by adding a C₃-C₇ fatty acid (such as sorbic acid or the like) to the beta blocker containing ophthalmic solution (WO 99/22715 A). In addition, the effect of extending the duration of action is obtained in the case where a composition prepared by adding a beta blocker to an aqueous solution of alginic acid and adjusting the resultant composition to pH 6 to 8 is applied to the eyes (JP 2002-511430 A).

The above-mentioned methods have already been applied to timolol maleate ophthalmic solutions and carteolol hydrochloride ophthalmic solutions. The above-mentioned methods demonstrate that once daily administration can produce the same effect as obtained by the twice daily administration of general aqueous ophthalmic solutions.

Generally, mannitol is well known as a typical sugar alcohol that finds a use as an isotonizing agent for controlling the osmotic pressure in consideration of the influence of ophthalmic solution after application. In addition, boric acid or borates are also generally used as a buffer or a preservative capable of exhibiting an antimicrobial action to some extent. It is also known that the ophthalmic solution containing a boric acid - polyol complex made of boric acid and a polyol including sugar alcohol can be provided with higher antimicrobial action against microorganisms including A. niger and the like than the ophthalmic solution containing boric acid alone, and the above-mentioned complex is practically used for the medicaments because of the ability to improve the preservation of aqueous compositions (WO 93/21903 A). However, any other effect than the antimicrobial action improving effect by the combined use of boric acid and a sugar alcohol has not been known.

As previously described, the improvement in the corneal permeability of beta blockers can reduce the dose of medicinal agent, for example, by decreasing the frequency of administration. It is expected that the systemic side effects will be reduced. In addition, less frequency of administration can improve the QOL and prevent the decrease of therapeutic effect which may be caused by missing the application to the eyes. In light of the above, the improvement in the corneal permeability of medicinal agent is extremely significant.

### [Summary of Invention]

### [Technical Problem]

An object of the invention is to provide an aqueous ophthalmic composition with improved corneal permeability of a medicinal agent for ophthalmic use, such as a beta blocker or the like.

### [Solution to Problem]

The inventors of the present invention have extensively studied about the ophthalmic solutions having an effect of enhancing the corneal permeability of therapeutic agents for glaucoma, in particular, beta blockers. As a result, it has been found that the above-mentioned problem can be solved by adding to the beta blocker-containing ophthalmic solution a sugar alcohol such as mannitol or the like, which the sugar alcohol can be used in many ways as an agent for adjusting the osmotic pressure of ophthalmic solution, and can be used at high concentrations because of the high stability and safety.

Accordingly, the invention provides an aqueous ophthalmic composition, comprising (A) a beta blocker and (B) a sugar alcohol.

It has also been found that the corneal permeability of the beta blocker can be increased by further adding boric acid to the above-mentioned aqueous ophthalmic composition.

The invention also provides an aqueous ophthalmic composition, comprising timolol or a pharmaceutically acceptable salt thereof as a beta blocker (A), mannitol as a sugar alcohol (B), and boric acid (C), and not comprising gellan gum, xanthan gum, methyl cellulose or hydroxypropylmethyl cellulose.

The invention also provides a corneal permeability enhancing agent for beta blocker, comprising a sugar alcohol.

In addition, the invention provides a use of a sugar alcohol as a corneal permeability enhancing agent for manufacturing an aqueous ophthalmic composition containing a beta blocker.

### [Effects of Invention]

The aqueous ophthalmic composition according to the invention has an effect of improving the corneal permeability of a medicinal agent for ophthalmic use, such as a beta blocker or the like. Thus, the dose to be applied can be reduced, for example by decreasing the frequency of administration. As a result, it is expected that the systemic side effects caused by administration of the beta blocker, i.e., the risk of cardiotoxicity, respiratory toxicity or the like will be reduced. In addition, less frequency of administration can improve the QOL and prevent the decrease of therapeutic effect which may be caused by missing the application to the eyes.

### [Brief Description of the Drawing]

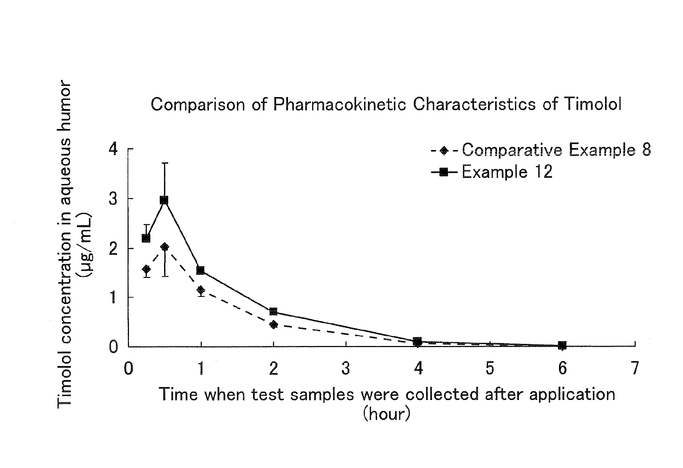

Fig. 1 is a graph showing the change in timolol concentration in the aqueous humor obtained by individually applying 30 µL of each sample substance to the eyes of white rabbits.

### [Description of Embodiments]

Examples of the beta blocker used as the component (A) in the invention include timolol, carteolol, nipradilol, betaxolol, levobunolol, and pharmaceutically acceptable salts thereof. The pharmaceutically acceptable salts are, for example, maleate, hydrochloride and the like. Of the above, timolol, carteolol, nipradilol or pharmaceutically acceptable salts thereof are preferable as the beta blocker.

The concentration of the beta blocker is not particularly limited so long as the intraocular pressure reducing effect can be obtained. The beta blocker may typically be used at a concentration of 0.01 to 10 w/v%, preferably 0.05 to 5 w/v%, and more preferably 0.1 to 3 w/v% when in a free form.

In addition to the beta blocker, medicinal agents such as prostaglandin derivatives including isopropyl unoprostone, bimatoprost, latanoprost, travoprost, tafluprost and the like may be contained as the component (D) in the composition of the invention.

Particularly, latanoprost and travoprost are preferable, and latanoprost is more preferable.

The content of the above-mentioned medicinal agent (D) is not particularly limited so long as the effects can be obtained, but may be generally in the range of 0.0001 to 10 w/v%, preferably 0.0005 to 5 w/v%, and more preferably 0.001 to 5 w/v%.

The composition of the invention may further comprise the following medicinal agents: nonselective adrenergic agonists such as dipivefrin hydrochloride, epinephrine and the like; α2-receptor selective adrenergic agonists such as brimonidine, apaclonidine hydrochloride and the like; parasympathetic agonists such as pilocarpine hydrochloride, distigmine bromide and the like; anti-infective agents such as amphotericin B, fluconazole, miconazole nitrate, colistin sodium methanesulfonate, carbenicillin sodium, gentamycin sulfate, erythromycin, azithromycin, tobramycin, kanamycin, ciprofloxacin hydrochloride, lomefloxacin hydrochloride, ofloxacin, levofloxacin, pazufloxacin tosylate, gatifloxacin, moxifloxacin hydrochloride, aciclovir, ganciclovir, cidofovir, sorivudine, trifluorothymidine, tetracyclines including doxycycline, and the like; antiallergic agents such as acitazanolast, levocarbastine hydrochloride, ketotifen fumarate, sodium cromoglycate, tranilast, olopatadine and the like; anti-inflammatory agents such as betamethasone phosphate, dexamethasone, hydrocortisone, diclophenac sodium, pranoprofen, indomethacin, bromfenac sodium, meloxicam, lornoxicam, cyclosporin, tacrolimus and the like; and therapeutic agents for cornea diseases and dry eye, such as aminoethylsulfonic acid, tetracyclines including doxycycline, and the like.

The content of the above-mentioned medicinal agents is not particularly limited so long as the effects can be obtained, but usually may be in the range of 0.001 to 10 w/v%.

Any sugar alcohols can be used with no limitation as the component (B) in the invention so long as the effects can be obtained. Mannitol, sorbitol and xylitol are preferable, and mannitol is particularly preferable. The concentration of the sugar alcohol is typically in the range of 0.01 to 10 w/v%, preferably 0.1 to 7 w/v%, more preferably 1 to 4 w/v%, and most preferably 1.5 to 4 w/v%.

The pharmaceutically acceptable salts of boric acid which may be used as the component (C) in the invention include borax, sodium salts and the like. Boric acid or the pharmaceutically acceptable salt thereof may be used at any concentrations so long as the corneal permeability of the medicinal agents can be enhanced. Typically, the content of boric acid may be in the range of 0.1 to 4 w/v%, preferably 0.5 to 3 w/v%, more preferably 0.7 to 2 w/v%, and most preferably 0.85 to 2 w/v%.

In the composition of the invention, the concentration ratio between the component (B) and the component (C) may preferably be in the range of 0.1 to 10, more preferably 0.5 to 5, and most preferably 1 to 4 when expressed as the ratio of the concentration (w/v%) of the component (B) to the concentration (w/v%) of the component (C).

The pH of the aqueous ophthalmic composition according to the invention may be 4.5 to 9.0, preferably 5.5 to 8.5, more preferably 6.0 to 8.0, and most preferably 6.7 to 8.0. A variety of pH adjustors commonly added may be used to adjust the pH value of the aqueous ophthalmic composition according to the invention. It is possible to use acids, for example, ascorbic acid, hydrochloric acid, glucuronic acid, acetic acid, lactic acid, phosphoric acid, sulfuric acid, citric acid, tartaric acid and the like. Bases such as borax, potassium hydroxide, calcium hydroxide, sodium hydroxide, magnesium hydroxide, monoethanolamine, diethanolamine, triethanolamine, trometamol, meglumine and the like can also be used. Examples of other pH adjustors include amino acids such as glycine, histidine, epsilon aminocaproic acid and the like.

In preparation of the aqueous ophthalmic composition of the invention, pharmaceutically acceptable isotonizing agents, solubilizers, stabilizers, preservatives and the like nlay be when necessary, so long as the effects of the invention are not impaired.

Examples of the isotonizing agent include propylene glycol, glycerin, sodium chloride, potassium chloride and the like. Examples of the solubilizer include polysorbate 80, polyoxyethylene hardened castor oil and the like.

Examples of the preservative include cationic soaps such as benzalkonium chloride, benzethonium chloride, chlorhexidine gluconate and the like; parabens such as methyl para-hydroxybenzoate, propyl para-hydroxybenzoate, butyl para-hydroxybenzoate and the like; alcohols such as chlorobutanol, phenylethyl alcohol, benzyl alcohol and the like; sodium dehydroacetate and the like.

Examples of the stabilizer include ethylenediaminetetraacetic acid and the pharmaceutically acceptable salts thereof, tocopherol and the derivatives thereof, sodium sulfite and the like.

Although the composition of the invention may comprise a thickener at low concentrations, the compositions that can cause sol-gel transition by the application of heat or the change of pH are not included in the present invention.

Even though certain polymers are added to the composition of the invention to such an extent that the sol-gel transition does not occur, but the viscosity of the resultant composition somewhat increases, the addition of the polymers is not intended to exhibit any synergy effect in the invention. The effects of the invention can be achieved without the addition of such polymers.

The thickener such as hydroxyethyl cellulose, polyvinyl pyrrolidone, polyethylene glycol and the like may be used, but the resultant composition of the invention may have a viscosity at 20°C of 1 to 15 mPa•s, preferably 1 to 10 mPa•s, more preferably 1 to 5 mPa•s, and most preferably 1 mPa•s.

The thickener may be added at a concentration of up to 0.5 w/v%. However, the composition of the invention can achieve the effect of enhancing the corneal permeability of the beta blocker without rendering viscosity by the addition of the thickener. Thus, it is preferable that no thickener be added. The composition not containing any thickener does not get sticky, thereby desirably leading to easy handling.

The composition of the invention does not comprise a thickener such as methyl cellulose, hydroxypropylmethyl cellulose (hypromellose), gellan gum, xanthan gum, carboxyvinyl polymer, sodium polyacrylate, sodium hyaluronate, polyvinyl alcohol, alginic acid or the like.

The osmotic pressure ratio of the aqueous ophthalmic composition according to the invention is not particularly limited so long as the effects of the invention can be obtained, but may typically be in the range of 0.5 to 2.0, preferably 0.7 to 1.6, more preferably 0.8 to 1.3, and most preferably 0.9 to 1.2.

The method for preparing the aqueous ophthalmic composition according to the invention will now be described by way of example.

A beta blocker and a sugar alcohol are added to sterile purified water together with a variety of additives, followed by sufficient stirring. After it is confirmed that all the components are dissolved to obtain a transparent solution, the pH is adjusted and the above-mentioned solution is diluted with sterile purified water. The aqueous solution thus obtained is subjected to filtration sterilization through a membrane filter, and then charged into a plastic bottle for eyedrops.

A beta blocker, a sugar alcohol, and boric acid or a pharmaceutically acceptable salt thereof are added to sterile purified water together with a variety of additives, followed by sufficient stirring. After it is confirmed that all the components are dissolved to obtain a transparent solution, the pH is adjusted and the above-mentioned solution is diluted with sterile purified water. The aqueous solution thus obtained is subjected to filtration sterilization through a membrane filter, and then charged into a plastic bottle for eyedrops.

According to a preferable embodiment of the invention, an aqueous composition comprising timolol maleate, mannitol, boric acid, benzalkonium chloride, and latanoprost may be adjusted to pH 6.7 to 8.0 and then charged into a plastic bottle for eyedrops to prepare an aqueous ophthalmic composition.

According to a particularly preferable embodiment of the invention, an aqueous composition comprising 0.68 w/v% of timolol maleate, 2.0 w/v% of mannitol, 1.0 w/v% of boric acid, benzalkonium chloride, and latanoprost may be adjusted to pH 6.7 with sodium hydrochloride, and then charged into a plastic bottle for eyedrops to prepare an aqueous ophthalmic composition.

### Examples

### [Example 1]

To 70 mL of sterile purified water, 0.68 g of timolol maleate, 1.5 g of mannitol, 0.85 g of boric acid, 1.0 g of sodium citrate and 0.2 mL of 0.5 w/v% benzalkonium chloride were added and dissolved in the water. 1N sodium hydroxide was used to adjust the above-mentioned aqueous solution to pH 6.9. Then, the aqueous solution was diluted with sterile purified water up to 100 mL. The aqueous solution thus obtained was subjected to filtration sterilization through a membrane filter, and 5 mL of the filtrate was charged into a plastic bottle for eyedrops. Thus, an aqueous ophthalmic composition was prepared in Example 1.

### [Comparative Example 1]

To 70 mL of sterile purified water, 0.68 g of timolol maleate, 1.39 g of boric acid, 1.0 g of sodium citrate and 0.2 mL of 0.5 w/v% benzalkonium chloride were added and dissolved in the water. 1N sodium hydroxide was used to adjust the above-mentioned aqueous solution to pH 6.9. Then, the aqueous solution was diluted with sterile purified water up to 100 mL. The aqueous solution thus obtained was subjected to filtration sterilization through a membrane filter, and 5 mL of the filtrate was charged into a plastic bottle for eyedrops. Thus, an aqueous ophthalmic composition was prepared in Comparative Example 1.

### [Comparative Example 2]

A commercially available 'TIMOPTOL (registered trademark) ophthalmic solution 0.5%' (made by Banyu Pharmaceutical Co., Ltd.) was prepared as a comparative ophthalmic solution in Comparative Example 2. This formulation contains as the additives a benzalkonium chloride solution, sodium dihydrogen phosphate, sodium hydrogen phosphate hydrate, and sodium hydroxide, and shows pH 6.9.

**[Table 1]**

| Components (w/v%) | Example 1 | Comparative Example 1 |
|---|---|---|
| Timolol maleate | 0.68 | 0.68 |
| Mannitol | 1.5 | - |
| Boric acid | 0.85 | 1.39 |
| Sodium citrate | 1.0 | 1.0 |
| Benzalkonium chloride | 0.001 | 0.001 |
| 1N NaOH | ad | ad |
| Sterile purified water (to 100 mL) | ad | ad |
| pH | 6.9 | 6.9 |

### <Test Example 1>

Using Japanese white rabbits (male, with weights of 2.0 to 2.5 kg), 50 µL of each ophthalmic solution prepared in Example 1, Comparative Example 1 or Comparative Example 2 was applied to the eye. Different ophthalmic solutions were applied to the left and right eyes of each rabbit and every ophthalmic solution was tested using six eyes (n=6). The aqueous humor was collected one hour after administration, and the timolol concentration in the aqueous humor was determined by HPLC.

Table 1 shows the formulations of the ophthalmic solutions, and Table 2 shows the timolol concentrations in the aqueous humor one hour after administration (average concentration each obtained from the data of the six eyes) and the ratio of timolol concentration in aqueous humor of each composition to that of Comparative Example 1 or 2.

**[Table 2]**

| | Example 1 | Comparative Example 1 | Comparative Example 2 'TIMOPTOL (Registered Trademark) ophthalmic solution 0.5%' |
|---|---|---|---|
| Drug concentration in aqueous humor (µg/mL) | 3.476 | 1.988 | 1.759 |
| Ratio of drug concentration in aqueous humor to that of Comparative Example 1 | 1.7 | 1.0 | 0.9 |
| Ratio of drug concentration in aqueous humor to that of Comparative Example 2 | 2.0 | 1.1 | 1.0 |

The composition of Comparative Example 1 did not contain mannitol, but contained boric acid, and the drug concentration in aqueous humor according to Comparative Example 1 was found to be similar to that according to Comparative Example 2. In the case where the composition of the invention comprising mannitol and boric acid was used, however, the drug concentration of aqueous humor was about 1.7 to 2 times higher than that of Comparative Example 2 or 1.

These results demonstrate that addition of mannitol and boric acid to the timolol maleate containing ophthalmic solution can significantly improve the corneal permeability of timolol.

### [Example 2]

To 70 mL of sterile purified water, 2.0 g of carteolol hydrochloride, 2.0 g of mannitol, 0.7 g of boric acid, 1.0 g of sodium citrate and 0.5 g of hydroxyethyl cellulose were added and dissolved in the water. 1N sodium hydroxide was used to adjust the above-mentioned aqueous solution to pH 6.9. Then, the aqueous solution was diluted with sterile purified water up to 100 mL. The aqueous solution thus obtained was subjected to filtration sterilization through a membrane filter, and 5 mL of the filtrate was charged into a plastic bottle for eyedrops. Thus, an aqueous ophthalmic composition was prepared in Example 2.

### [Example 3]

To 70 mL of sterile purified water, 2.0 g of carteolol hydrochloride, 3.9 g of mannitol, 1.0 g of sodium citrate, 0.5 g of hydroxyethyl cellulose, and 0.2 mL of 0.5 w/v% benzalkonium chloride were added and dissolved in the water. 1N sodium hydroxide was used to adjust the above-mentioned aqueous solution to pH 7.9. Then, the aqueous solution was diluted with sterile purified water up to 100 mL. The aqueous solution thus obtained was subjected to filtration sterilization through a membrane filter, and 5 mL of the filtrate was charged into a plastic bottle for eyedrops. Thus, an aqueous ophthalmic composition was prepared in Example 3.

### [Comparative Example 3]

A commercially available 'Mikelan (registered trademark) ophthalmic solution 2%' ( made by Otsuka Pharmaceutical Co., Ltd.) was prepared as a comparative ophthalmic solution in Comparative Example 3. This ophthalmic solution contains as the additives a benzalkonium chloride solution, sodium chloride, sodium dihydrogen phosphate, and disodium hydrogen phosphate anhydrous. The ophthalmic solution of Comparative Example 3 had a viscosity at 20°C of 1 mPa•s.

**[Table 3]**

| Components (w/v%) | Example 2 | Example 3 |
|---|---|---|
| Carteolol hydrochloride | 2.0 | 2.0 |
| Mannitol | 2.0 | 3.9 |
| Boric acid | 0.7 | - |
| Sodium citrate | 1.0 | 1.0 |
| Hydroxyethyl cellulose | 0.5 | 0.5 |
| Benzalkonium chloride | - | 0.00 |
| 1N NaOH | ad | ad |
| Sterile purified water (to 100 mL) | ad | ad |
| pH | 6.9 | 7.9 |
| Viscosity at 20°C (mPa•s) | 3 | 3 |

### <Test Example 2>

Using Japanese white rabbits (male, with weights of 2.0 to 2.5 kg), 30 µL of each ophthalmic solution prepared in Example 2 or 3, or Comparative Example 3 was applied to the eye. Different ophthalmic solutions were applied to the left and right eyes of each rabbit and every ophthalmic solution was tested using six eyes (n=6). The aqueous humor was collected 1.5 hours after administration, and the carteolol concentration in the aqueous humor was determined by HPLC.

Table 3 shows the formulations of the ophthalmic solutions, and Table 4 shows the carteolol concentrations in the aqueous humor 1.5 hours after administration (average concentration each obtained from the data of the six eyes) and the ratio of carteolol concentration in aqueous humor of each composition to that of Comparative Example 3.

**[Table 4]**

| | Example 2 | Example 3 | Comparative Example 3 'Mikelan (Registered Trademark) ophthalmic solution 2%' |
|---|---|---|---|
| Drug concentration in aqueous humor (µg/mL) | 1.660 | 1.191 | 0.807 |
| Ratio of drug concentration in aqueous humor to that of Comparative Example 3 | 2.1 | 1.5 | 1.0 |

The carteolol concentrations in aqueous humor obtained by the mannitol-containing compositions according to the invention were 1.5 to 2.1 times higher than that according to Comparative Example 3. Further, the corneal permeability of carteolol according to Example 2 where both boric acid and mannitol were used was found to be higher than that according to Example 3 where the amount of mannitol used was about twice, but boric acid was not used.

These results demonstrate that addition of mannitol alone or in combination with boric acid to the carteolol hydrochloride containing ophthalmic solution can significantly improve the corneal permeability of carteolol.

### [Example 4]

To 70 mL of sterile purified water, 0.25 g ofnipradilol, 3.9 g of mannitol, 1.0 g of sodium citrate, 0.5 g of hydroxyethyl cellulose, and 0.2 mL of 0.5 w/v% benzalkonium chloride were added and dissolved in the water. 1N sodium hydroxide was used to adjust the above-mentioned aqueous solution to pH 7.9. Then, the aqueous solution was diluted with sterile purified water up to 100 mL. The aqueous solution thus obtained was subjected to filtration sterilization through a membrane filter, and 5 mL of the filtrate was charged into a plastic bottle for eyedrops. Thus, an aqueous ophthalmic composition was prepared in Example 4.

### [Comparative Example 4]

A commercially available 'HYPADIL (registered trademark) Kowa (registered trademark) ophthalmic solution 0.25%' ( made by Kowa Pharmaceutical Co., Ltd.) was prepared as a comparative ophthalmic solution in Comparative Example 4. This ophthalmic solution contains as the additives sodium hydrogen phosphate, potassium dihydrogen phosphate, hydrochloric acid, sodium chloride, and a benzalkonium chloride solution. The ophthalmic solution of Comparative Example 4 had a viscosity at 20°C of 1 mPa•s.

**[Table 5]**

| Components (w/v%) | Example 4 |
|---|---|
| Nipradilol | 0.25 |
| Mannitol | 3.9 |
| Sodium citrate | 1.0 |
| Hydroxyethyl cellulose | 0.5 |
| Benzalkonium chloride | 0.001 |
| 1N NaOH | ad |
| Sterile purified water (to 100 mL) | ad |
| pH | 7.9 |
| Viscosity at 20°C (mPa•s) | 3 |

### <Test Example 3>

Using Japanese white rabbits (male, with weights of 2.0 to 2.5 kg), 30 µL of each ophthalmic solution according to Example 4 or Comparative Example 4 was applied to the eye. Different ophthalmic solutions were applied to the left and right eyes of each rabbit and each ophthalmic solution was tested using six eyes (n=6). The aqueous humor was collected 0.5 hours after administration, and the nipradilol concentration in the aqueous humor was determined by HPLC.

Table 5 shows the formulations of the ophthalmic solution, and Table 6 shows the nipradilol concentrations in the aqueous humor 1.5 hours after administration (average concentration each obtained from the data of the six eyes) and the ratio of the nipradilol concentration in aqueous humor of the composition according to the invention to that of 'HYPADIL (registered trademark) Kowa (registered trademark) ophthalmic solution 0.25%'.

**[Table 6]**

| | Example 4 | Comparative Example 4 'HYPADIL (Registered Trademark) Kowa (Registered Trademark) ophthalmic solution 0.25%' |
|---|---|---|
| Drug concentration in aqueous humor (µg/mL) | 0.392 | 0.336 |
| Ratio of drug concentration in aqueous humor to that of Comparative Example 4 | 1.2 | 1.0 |

The nipradilol concentration in aqueous humor obtained by the mannitol-containing composition according to the invention was 1.2 times higher than that according to Comparative Example 4.

This result demonstrates that addition of mannitol to the nipradilol containing ophthalmic solution can improve the corneal permeability of nipradilol.

### [Example 5]

To 70 mL of sterile purified water, 2.0 g of carteolol hydrochloride, 2.0 g of mannitol and 1.0 g of boric acid were added and dissolved in the water. 1N sodium hydroxide was used to adjust the above-mentioned aqueous solution to pH 6.9. Then, the aqueous solution was diluted with sterile purified water up to 100 mL. The aqueous solution thus obtained was subjected to filtration sterilization through a membrane filter, and 5 mL of the filtrate was charged into a plastic bottle for eyedrops. Thus, an aqueous ophthalmic composition was prepared in Example 5.

### [Comparative Example 5]

A commercially available 'Mikelan (registered trademark) LA ophthalmic solution 2%' (made by Senju Pharmaceutical Co., Ltd.) was prepared as a comparative ophthalmic solution in Comparative Example 5. This ophthalmic solution contains as the additives a benzalkonium chloride solution, sodium chloride, sodium dihydrogen phosphate, disodium hydrogen phosphate anhydrous, sodium hydroxide and alginic acid.

**[Table 7]**

| Components (w/v%) | Example 5 |
|---|---|
| Carteolol hydrochloride | 2.0 |
| Mannitol | 2.0 |
| Boric acid | 1.0 |
| 1N NaOH | ad |
| Sterile purified water (to 100 mL) | ad |
| pH | 6.9 |

### <Test Example 4>

Using Japanese white rabbits (male, with weights of 2.0 to 2.5 kg), 30 µL of each ophthalmic solution according to Example 5 or Comparative Example 5 was applied to the eye. Different ophthalmic solutions were applied to the left and right eyes of each rabbit and each ophthalmic solution was tested using six eyes (n=6). The aqueous humor was collected one and two hours after administration, and the carteolol concentration in the aqueous humor was determined by HPLC.

Table 7 represents the formulation of the ophthalmic solution, and Table 8 represents the carteolol concentrations in the aqueous humor one and two hours after administration (average concentration each obtained from the data of the six eyes) and the ratio of the carteolol concentration in aqueous humor of the composition according to the invention to that of 'Mikelan (registered trademark) LA ophthalmic solution 2%'.

**[Table 8]**

| | Example 5 | Comparative Example 5 'Mikelan (Registered Trademark) LA ophthalmic solution 2%' |
|---|---|---|
| Drug concentration in aqueous humor after one hour (µg/mL) | 1.436 | 0.937 |
| Ratio of drug concentration in aqueous humor after one hour to that of Comparative Example 5 | 1.5 | 1.0 |
| Drug concentration in aqueous humor after two hours (µg/mL) | 1.216 | 0.860 |
| Ratio of drug concentration in aqueous humor after two hours to that of Comparative Example 5 | 1.4 | 1.0 |

In contrast to 'Mikelan (registered trademark) ophthalmic solution 2%' of Comparative Example 3, which is designed to be administered twice a day, 'Mikelan (registered trademark) LA ophthalmic solution 2%' of Comparative Example 5 is formulated as a long acting drug and commercially available as an ophthalmic solution designed to be administered once a day although the content of the medicinal component is equal.

When compared with the comparative composition of Comparative Example 5, the composition comprising mannitol and boric acid according to the invention showed carteolol concentrations in aqueous humor 1.4 to 1.5 times higher than those of the composition of Comparative Example 5.

This result suggests that the addition of mannitol and boric acid to the carteolol containing ophthalmic solution can improve the corneal permeability of carteolol, and keep high drug concentrations in the ocular tissue over a long period of time.

### [Examples 6 to 9]

To 70 mL of sterile purified water, 2.0 g of carteolol hydrochloride, and predetermined amounts of mannitol and boric acid were added and dissolved in the water. 1N sodium hydroxide was used to adjust the aqueous solution to pH 6.9. Then, the aqueous solution was diluted with sterile purified water up to 100 mL. The aqueous solution thus obtained was subjected to filtration sterilization through a membrane filter, and 5 mL of the filtrate was charged into a plastic bottle for eyedrops. Thus, aqueous compositions for ophthalmic solution were prepared in Example 6 to 9 as shown in Table 9.

### [Comparative Example 6]

To 70 mL of sterile purified water, 2.0 g of carteolol hydrochloride and 1.2 g of boric acid were added and dissolved in the water. 1N sodium hydroxide was used to adjust the above-mentioned aqueous solution to pH 6.9. Then, the aqueous solution was diluted with sterile purified water up to 100 mL. The aqueous solution thus obtained was subjected to filtration sterilization through a membrane filter, and 5 mL of the filtrate was charged into a plastic bottle for eyedrops. Thus, an aqueous ophthalmic composition was prepared in Comparative Example 6 as shown in Table 9.

**[Table 9]**

| Components (w/v%) | Ex. 6 | Ex. 7 | Ex. 8 | Ex. 9 | Comp. Ex. 6 |
|---|---|---|---|---|---|
| Carteolol hydrochloride | 2.0 | 2.0 | 2.0 | 2.0 | 2.0 |
| Mannitol | 2.0 | 2.0 | 1.0 | 4.0 | - |
| Boric acid | 1.0 | 0.7 | 1.0 | 2.0 | 1.2 |
| 1N NaOH | ad | ad | ad | ad | ad |
| Sterile purified water (to 100 mL) | ad | ad | ad | ad | ad |
| pH | 6.9 | 6.9 | 6.9 | 6.9 | 6.9 |

### <Test Example 5>

Using Japanese white rabbits (male, with weights of 2.0 to 2.5 kg), 30 µL of each ophthalmic solution according to Examples 6 to 10 or Comparative Example 6 was applied to the eye. Different ophthalmic solutions were applied to the left and right eyes of each rabbit and every ophthalmic solution was tested using six eyes (n=6). The aqueous humor was collected 1.5 hours after administration, and the carteolol concentration in the aqueous humor was determined by HPLC.

Table 9 shows the formulations of the ophthalmic solutions, and Table 10 shows the carteolol concentrations in the aqueous humor 1.5 hours after administration (average concentration each obtained from the data of the six eyes) of the ophthalmic solutions according to Examples 6 to 10 and Comparative Example 6, and the ratio of the carteolol concentration in aqueous humor of each composition according to the invention to that of Comparative Example 6.

**[Table 10]**

| | Drug concentration in aqueous humor (µg/mL) | Ratio of drug concentration in aqueous humor to that of Comparative Example 6 |
|---|---|---|
| Example 6 | 1.424 | 1.4 |
| Example 7 | 1.250 | 1.2 |
| Example 8 | 1.352 | 1.3 |
| Example 9 | 1.353 | 1.3 |
| Comparative Example 6 | 1.061 | 1.0 |

In contrast to the composition just containing 1.2 w/v% of boric acid according to Comparative Example 6, the compositions of the invention containing mannitol in an amount ranging from 1.0 to 4.0 w/v% and boric acid in an amount ranging from 0.7 to 2.0 w/v% showed higher carteolol concentrations in aqueous humor. Especially, the compositions of the invention containing mannitol in an amount ranging from 1.0 to 4.0 w/v% and boric acid in an amount ranging from 1.0 to 2.0 w/v% showed particularly higher carteolol concentrations in aqueous humor.

### [Example 10]

0.2 g of benzalkonium chloride was added to 700 mL of sterile purified water and dissolved therein to prepare an aqueous solution. Next, 0.05 g of latanoprost was further added and dissolved in the aqueous solution. Then, 6.8 g of timolol maleate, 20.0 g of mannitol and 10.0 g of boric acid were added to the aqueous solution and dissolved therein. 1N sodium hydroxide was used to adjust the above-mentioned aqueous solution to pH 6.7. Then, the aqueous solution was diluted with sterile purified water up to 1000 mL. The aqueous solution thus obtained was subjected to filtration sterilization through a membrane filter, and 5 mL of the filtrate was charged into a plastic bottle for eyedrops. Thus, an aqueous ophthalmic composition was prepared in Example 10.

### [Comparative Example 8]

0.2 g of benzalkonium chloride was added to 700 mL of sterile purified water and dissolved therein to prepare an aqueous solution. Next, 0.05 g of latanoprost was further added and dissolved in the aqueous solution. Then, 6.8 g of timolol maleate, 3.6 g of sodium chloride, and 10.0 g of boric acid were added to the aqueous solution and dissolved therein. 1N sodium hydroxide was used to adjust the above-mentioned aqueous solution to pH 6.8. Then, the aqueous solution was diluted with sterile purified water up to 1000 mL. The aqueous solution thus obtained was subjected to filtration sterilization through a membrane filter, and 5 mL of the filtrate was charged into a plastic bottle for eyedrops. Thus, an aqueous ophthalmic composition was prepared in Comparative Example 8.

**[Table 11]**

| Components (w/v%) | Example 10 | Comparative Example 8 |
|---|---|---|
| Latanoprost | 0.005 | 0.005 |
| Timolol maleate | 0.68 | 0.68 |
| Mannitol | 2.0 | - |
| Boric acid | 1.0 | 1.0 |
| Sodium chloride | - | 0.36 |
| Benzalkonium chloride | 0.02 | 0.02 |
| 1N NaOH | ad | ad |
| Sterile purified water (to 100 mL) | ad | ad |
| pH | 6.7 | 6.8 |

### <Test Example 7>

Using Japanese white rabbits (male, with weights of 2.0 to 2.5 kg), 30 µL of each ophthalmic solution prepared in Example 10 or Comparative Example 8 was applied to the eye. Different ophthalmic solutions were applied to the left and right eyes of each rabbit and each ophthalmic solution was tested using five eyes (n=5) every time the aqueous humor was collected. The aqueous humor was collected 15 minutes, 30 minutes, one hour, two hours, four hours, and six hours after administration, and the timolol concentration in the aqueous humor was determined by LC/MS/MS.

Table 11 shows the formulations of the ophthalmic solutions. Table 12 shows the pharmacokinetic parameters calculated from the pharmacokinetic characteristics of timolol, i.e., the timolol concentrations in the aqueous humor (average concentration each obtained from the data of the five eyes) 15 minutes, 30 minutes, one hour, two hours, four hours, and six hours after administration; and the ratio of each parameter value obtained from the composition of Example 10 according to the invention to that from the composition of Comparative Example 8. Fig. 1 is a diagram representing the pharmacokinetics.

**[Table 12]**

| Test Samples | Tₘₐₓ | Cmax | AUC₀₋₆ₕᵣ | AUC_{∞} | T_{1/2} |
|---|---|---|---|---|---|
| | (hr) | (µg/mL) | (µg/mL•hr) | (µg/mL•hr) | (hr) |
| Comparative Example 8 | 0.5 | 2.017 | 2.847 | 2.854 | 0.7 |
| Example 10 | 0.5 | 2.958 | 4.092 | 4.120 | 0.8 |
| Ex.10/Comp. Ex. 8 | - | 1.5 | 1.4 | 1.4 | - |

As can be seen from the results of Table 12, the parameter values of Cₘₐₓ and AUC_{∞} of the composition of Example 10 comprising mannitol and boric acid according to the invention were respectively 1.5 times and 1.4 times higher than those of Comparative Example 8. Further, the timolol concentration in aqueous humor obtained by the composition of Example 10 was found to consistently keep higher than that of Comparative Example 8 through six hours after administration.

The above-mentioned results show that the composition of the invention comprising the prostaglandin derivative in addition to the beta blocker as the active ingredients together with mannitol and boric acid can keep the concentrations of the beta blocker in aqueous humor higher for a long period of time than the comparative composition not containing mannitol.

It is apparent that the effects of the invention can be exhibited whether the beta blocker is used alone or in combination with other agent.

### [Example 11]

To 70 mL of sterile purified water, 0.68 g of timolol maleate, 1.0 g of sorbitol, 1.2 g of boric acid, 1.0 g of sodium citrate and 0.2 mL of 0.5 w/v% benzalkonium chloride were added and dissolved in the water. 1N sodium hydroxide was used to adjust the above-mentioned aqueous solution to pH 6.9. Then, the aqueous solution was diluted with sterile purified water up to 100 mL. The aqueous solution thus obtained was subjected to filtration sterilization through a membrane filter, and 5 mL of the filtrate was charged into a plastic bottle for eyedrops. Thus, an aqueous ophthalmic composition was prepared according to the invention.

### [Example 12]

To 70 mL of sterile purified water, 0.25 g of nipradilol, 2.0 g of xylitol, 1.4 g of boric acid, and 0.2 mL of 0.5 w/v% benzalkonium chloride were added and dissolved in the water. 1N sodium hydroxide was used to adjust the above-mentioned aqueous solution to pH 7.0. Then, the aqueous solution was diluted with sterile purified water up to 100 mL The aqueous solution thus obtained was subjected to filtration sterilization through a membrane filter, and 5 mL of the filtrate was charged into a plastic bottle for eyedrops. Thus, an aqueous ophthalmic composition was prepared according to the invention.

### [Example 13]

To 70 mL of sterile purified water, 0.005 g of latanoprost and 0.5 g of polysorbate 80 were added and dissolved in the water to prepare an aqueous solution. To the aqueous solution, 0.68 g of timolol maleate, 2.0 g of mannitol, and 0.7 g of boric acid were further added and dissolved therein. 1N sodium hydroxide was used to adjust the above-mentioned aqueous solution to pH 6.7. Then, the aqueous solution was diluted with sterile purified water up to 100 mL. The aqueous solution thus obtained was subjected to filtration sterilization through a membrane filter, and 2.5 mL of the filtrate was charged into a plastic bottle for eyedrops. Thus, an aqueous ophthalmic composition was prepared according to the invention.

The aqueous solution was diluted with sterile purified water up to 100 mL. The aqueous solution thus obtained was subjected to filtration sterilization through a membrane filter, and 5 mL of the filtrate was charged into a plastic bottle for eyedrops. Thus, an aqueous ophthalmic composition was prepared according to the invention.

### Industrial Applicability

The corneal permeability of a medicinal agent contained in a formulation can be enhanced by adding a sugar alcohol to the formulation. Further addition of boric acid can lead to higher corneal permeability of the medicinal agent. When the corneal permeability of the beta blocker is improved, for example, the frequency of administration can be reduced, which makes it possible to decrease the dose of the drug. As a result, it is expected that the systemic side effects, such as the risk of cardiotoxicity, respiratory toxicity or the like will be reduced. In addition, less frequency of administration can improve the QOL and prevent the decrease of therapeutic effect which may be caused by missing the application to the eyes.

## Claims

1. An aqueous ophthalmic composition, comprising (A) a beta blocker and (B) a sugar alcohol.

2. The aqueous ophthalmic composition of claim 1, wherein (A) the beta blocker is at least one selected from the group consisting of timolol, carteolol, nipradilol, betaxolol, levobunolol, and pharmaceutically acceptable salts thereof.

3. The aqueous ophthalmic composition of claim 1 or 2, wherein (B) the sugar alcohol is at least one selected from the group consisting of mannitol, sorbitol and xylitol.

4. The aqueous ophthalmic composition of any one of claims 1 to 3, further comprising (C) boric acid or a pharmaceutically acceptable salt thereof.

5. The aqueous ophthalmic composition of any one of claims 1 to 4, further comprising (D) a prostaglandin derivative.

6. The aqueous ophthalmic composition of any one of claims 1 to 4, wherein (D) the prostaglandin derivative is latanoprost.

7. The aqueous ophthalmic composition of any one of claims 1 to 6, wherein (B) the sugar alcohol is mannitol.

8. The aqueous ophthalmic composition of claim 1, wherein (A) the beta blocker is carteolol or a pharmaceutically acceptable salt thereof, and (B) the sugar alcohol is mannitol. 1.

9. The aqueous ophthalmic composition of claim 1, wherein (A) the beta blocker is nipradilol or a pharmaceutically acceptable salt thereof, and (B) the sugar alcohol is mannitol.

10. An aqueous ophthalmic composition, comprising timolol or a pharmaceutically acceptable salt thereof as (A) a beta blocker, mannitol as (B) a sugar alcohol, and (C) boric acid, and not containing gellan gum, xanthan gum, methyl cellulose or hydroxypropylmethyl cellulose.

11. The aqueous ophthalmic composition of claim 10, further comprising latanoprost as (D) a prostaglandin derivative.

12. A corneal permeability enhancing agent for beta blocker, comprising a sugar alcohol.

13. A use of a sugar alcohol as a corneal permeability enhancing agent for manufacturing an aqueous ophthalmic composition comprising a beta blocker.
